# EUROPÄISCHE PATENTSCHRIFT

(11) **EP 2 211 987 B1**
(45) Veröffentlichungstag und Bekanntmachung des Hinweises auf die Patenterteilung: **24.07.2013**
(21) Anmeldenummer: 08844104.3
(22) Anmeldetag: 28.10.2008
(51) Int. Cl.: A61N 1/36, A61N 1/32, A61N 1/08, A61N 1/05

(54) **VORRICHTUNG ZUR STIMULATION VON NEURONENVERBÄNDEN**
APPARATUS FOR THE STIMULATION OF NEURAL NETWORKS
DISPOSITIF DE STIMULATION DE RESEAUX NEURONAUX

(30) Priorität: 30.10.2007 DE 102007051848
(43) Veröffentlichungstag der Anmeldung: 04.08.2010
(73) Patentinhaber: Forschungszentrum Jülich GmbH, 52425 Jülich (DE)
(72) Erfinder: TASS, Peter, 52445 Titz (DE); HAUPTMANN, Christian, 52224 Stolberg (DE)
(74) Vertreter: Manitz, Finsterwald & Partner GbR
(86) Internationale Anmeldenummer: PCT/DE2008/001746
(87) Internationale Veröffentlichungsnummer: WO 2009/056106

(56) Entgegenhaltungen:
- US-A1- 2003 028 072
- US-A1- 2006 224 191

## Beschreibung

Die Anmeldung betrifft eine Vorrichtung und ein Verfahren welches nicht Teil des Erfindung ist zur Stimulation von Neuronenverbänden. Insbesondere bezieht sich die Erfindung auf eine Vorrichtung zur Wiederherstellung der Aktivität von Rhythmusgeneratoren.

Rhythmusgeneratoren (englisch: central pattern generator; CPG) sind ein wichtiges funktionales Element des Zentralnervensystems und verantwortlich z.B. für die Steuerung und Initiierung von Bewegungen. Sie finden sich beispielsweise im Rückenmark sowie im Stammhirn. Rhythmusgeneratoren sind neuronale Netzwerke, die endogen, d.h. ohne äußere Anreize, durch das "Feuern" der beteiligten Neuronen sich rhythmisch wiederholende Muster erzeugen. Als "Feuern" eines Neuronen wird das Erzeugen eines kurzen, elektrischen Pulses bezeichnet, durch den an die mit dem feuernden Neuron in Verbindung stehenden Neuronen Signale übermittelt werden.

Ein Rhythmusgenerator setzt sich aus mehreren Neuronenverbänden zusammen, wobei die Neuronen innerhalb eines jeden Neuronenverbandes synchron aktiv sind, d.h. synchron feuern. Die Aktivität der Neuronenverbände untereinander ist zeitlich verschoben. Störungen der Aktivität von Rhythmusgeneratoren können beispielsweise zu starken motorischen Störungen führen.

Die Schriften US 2003/0028072 A1 und US 2006/0224191 A1 offenbaren jeweils Vorrichtungen mit Messeinheiten zum Aufnehmen von Messsignalen von Neuronen, Generatoreinheiten zum Erzeugen von elektrischen Stimulationssignalen in Abhängigkeit von den Messsignalen und Stimulationseinheiten zur Stimulation von Neuronenverbänden mit den Stimulationssignalen. In der Schrift US 2006/0224191 A1 sind die Stimulationssignale ferner als Pulszüge ausgestaltet, die mit einem aus den Messsignalen gewonnenem Modulationssignal moduliert sind.

Vor diesem Hintergrund wird die Erfindung gemäß der in Anspruch 1 angegeben en Vorrichtung definiert. Vorteilhafte Weiterbildungen und Ausgestaltungen der Erfindung sind in den Unteransprüchen angegeben.

Gemäß einer Ausgestaltung umfasst eine Vorrichtung mindestens eine Messeinheit, eine Generatoreinheit und eine Mehrzahl von Stimulationseinheiten, die an die Generatoreinheit gekoppelt sind. Mittels der Messeinheit werden Messsignale von Neuronen aufgenommen. Die Generatoreinheit dient zum Erzeugen von elektrischen Stimulationssignalen, die an die Stimulationseinheiten weitergeleitet werden. Dabei werden die Stimulationssignale in Abhängigkeit von den Messsignalen erzeugt. Die Stimulationseinheiten stimulieren mit den Stimulationssignalen jeweils unterschiedliche Neuronenverbände. Ferner applizieren die Stimulationseinheiten die Stimulationssignale jeweils zeitversetzt, z.B. beginnt jede der Stimulationseinheiten mit der Stimulation zu einem anderen Zeitpunkt. Durch die zeitversetzte Stimulation wird in die stimulierten Neuronenverbände eine zeitlich verschobene Aktivität induziert.

Gemäß einer weiteren Ausgestaltung der Vorrichtung können auch Stimulationssignale mit unterschiedlicher Polarität ohne oder zusammen mit einem zeitlichen Versatz appliziert werden.

Die Vorrichtung kann zur Wiederherstellung der Aktivität von Rhythmusgeneratoren verwendet werden.

Ferner kann die Vorrichtung zur Behandlung nach einem Schlaganfall, bei der Erkrankung "Gait-Ignition-Disorder" oder einer anderen motorischen Störung verwendet werden.

Die Erfindung wird nachfolgend in beispielhafter Weise unter Bezugnahme auf die Zeichnungen näher erläutert. In diesen zeigen:
- Fig. 1: eine schematische Darstellung einer Vorrichtung 100 gemäß einem Ausführungsbeispiel;
- Fig. 2: eine schematische Darstellung einer Stimulations- und Messelektrode 200;
- Fig. 3: eine schematische Darstellung von mittels mehrerer Stimulationseinheiten applizierten Sequenzen von Stimulationssignalen 300;
- Fig. 4: eine schematische Darstellung einer mittels einer Stimulationseinheit applizierten Sequenz von Pulszügen 300;
- Fig. 5: eine schematische Darstellung von mittels mehrerer Stimulationseinheiten applizierten Sequenzen von Pulszügen 300;
- Fig. 6: eine schematische Darstellung eines Pulszugs 300;
- Fig. 7: eine schematische Darstellung von mittels mehrerer Stimulationseinheiten applizierten und aus Messsignalen gewonnenen Stimulationssignalen 700 sowie 701;
- Fig. 8: eine schematische Darstellung von mittels einer Messeinheit aufgenommenen Messsignalen 800;
- Fig. 9: eine schematische Darstellung von mittels mehrerer Stimulationseinheiten applizierten und aus Messsignalen gewonnenen Stimulationssignalen 900; und
- Fig. 10: eine schematische Darstellung einer Vorrichtung 1000 gemäß einem weiteren Ausführungsbeispiel.

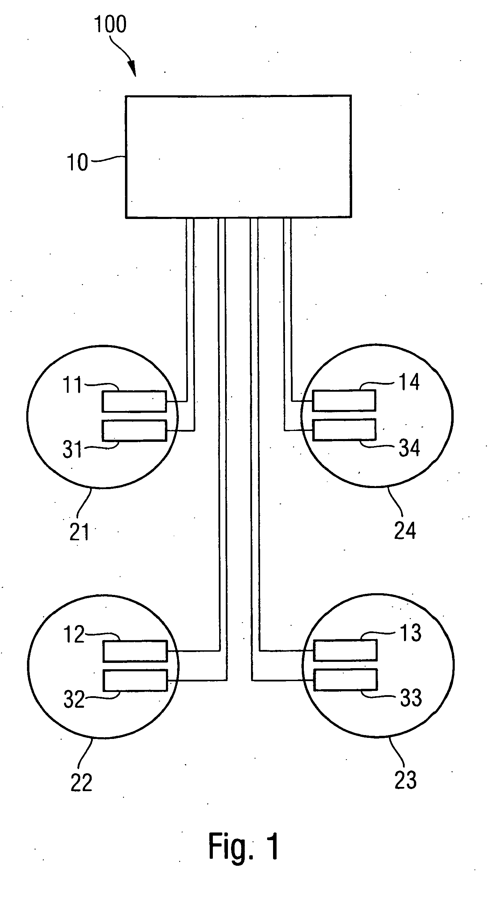

In Fig. 1 ist schematisch eine Vorrichtung 100 dargestellt. Die Vorrichtung 100 beinhaltet eine Generatoreinheit 10 und eine Mehrzahl von Stimulationseinheiten 11, 12, 13 sowie 14, die mit der Generatoreinheit 10 in Verbindung stehen. In dem vorliegenden Ausführungsbeispiel beträgt die Anzahl der Stimulationseinheiten vier, es können jedoch auch zwei, drei, fünf, sechs oder mehr Stimulationseinheiten vorgesehen sein.

Jede der Stimulationseinheiten 11 bis 14 ist derart im Gehirn oder im Bereich des Rückenmarks eines Menschen oder Tiers platziert, dass die betreffende Stimulationseinheit einen Neuronenverband 21, 22, 23 bzw. 24 stimuliert, d.h. jede Stimulationseinheit 11 bis 14 ist einem der Neuronenverbände 21 bis 24 zugeordnet. Die Neuronenverbände 21 bis 24 können Teil eines zentralen Rhythmusgenerators sein.

Während des Betriebs der Vorrichtung 100 erzeugt die Generatoreinheit 10 Stimulationssignale, die in die Stimulationseinheiten 11 bis 14 eingespeist werden und von den Stimulationseinheiten 11 bis 14 zur Stimulation der Neuronenverbände 21 bis 24 verwendet werden. Dabei sind die von unterschiedlichen Stimulationseinheiten 11 bis 14 applizierten Stimulationssignale jeweils zeitversetzt.

Durch die mit Hilfe der Vorrichtung 100 durchführbare Neurostimulation kann jedem der Neuronenverbände 21 bis 24 eine rhythmische Aktivität induziert werden, wobei die rhythmischen Aktivitäten unterschiedlicher Neuronenverbände 21 bis 24 bzw. deren Phasen zeitlich gegeneinander verschoben sind. Die zeitlich verschobene rhythmische Aktivität der Neuronenverbände 21 bis 24 entspricht annähernd der normalen, gesunden Aktivität von zentralen Rhythmusgeneratoren. Dabei weisen die Neuronen innerhalb eines Neuronenverbands die gleiche Aktivität auf, d.h. die zu ein und demselben Neuronenverband gehörenden Neuronen feuern synchron. Bei einer geeigneten Wahl der Stimulationsstellen kann die Stimulation mittels der Vorrichtung 100 eingesetzt werden, um einer krankhaften Reduktion solcher rhythmischer Aktivität entgegenzuwirken. Dies führt zu einer Reduzierung der krankhaften Symptomatik.

Eine krankhafte Reduktion der rhythmischen Aktivität von Rhythmusgeneratoren kann beispielsweise nach einem Schlaganfall oder bei der Erkrankung "Gait-Ignition-Disorder" oder auch bei Morbus Parkinson beobachtet werden. Die Vorrichtung 100 kann zur Behandlung derartiger Erkrankungen eingesetzt werden.

Die Stimulation mittels der Vorrichtung 100 kann beispielsweise bewirken, dass die Neuronenverbände 21 bis 24 den Rhythmus und Zeitversatz annehmen, der ihnen durch die Stimulation vorgegeben wird, d.h. Rhythmus und Zeitversatz werden den stimulierten Neuronenverbänden durch die Stimulationssignale aufgeprägt. Bei Patienten, deren zentrale Rhythmusgeneratoren gestört sind, kann sich aufgrund der Stimulation eine langanhaltende oder gar dauerhafte Stabilisierung der gesunden Funktionsweise der Neuronenverbände einstellen.

Rhythmusgeneratoren befinden sich beim Menschen beispielsweise im Bereich des Rückenmarks. Neben diesen befinden sich neuronale Strukturen, die für die Erzeugung rhythmischer Aktivität zuständig sind, auch an anderen Stellen des zentralen Nervensystems, z.B. innerhalb des Gehirns. Im Rückenmark befinden sich beispielsweise Populationen sogenannter Interneuronen, die als Generatoren von koordinierter Bewegungsaktivität wirken. Beispielhaft kann eine solche Rhythmusgeneratorstruktur durch eine epidurale Stimulation an der dorsalen Oberfläche des Rückenmarks im Bereich der Lendenwirbelsäule realisiert werden. Bei Patienten mit einem Trauma der Wirbelsäule kann eine Wiederherstellung der rhythmischen Aktivität durch eine Stimulation der Rhythmusgeneratoren in diesem Bereich mittels der Vorrichtung 100 zu einer Initialisierung und Ausführung der Bewegung führen.

Die gleiche Wirkung wie bei der zeitversetzten Applikation der Stimulationssignale über die Stimulationseinheiten 11 bis 14 kann erzielt werden, wenn die Stimulationssignale zeitgleich, aber mit unterschiedlicher Polarität appliziert werden. Wird z.B. ein Sinus-Signal an zwei Stimulationsstellen zeitgleich und mit unterschiedlicher Polarität appliziert, so entspricht dies einer Stimulation mit einer Phasendifferenz von 180°.

Die Vorrichtung 100 enthält zusätzlich noch eine oder mehrere Messeinheiten 31 bis 34, welche Messsignale von Nervenzellen, beispielweise in Form von elektrischen Pulsen, aufnehmen und diese an die Generatoreinheit 10 weiterleiten. Die Generatoreinheit 10 erzeugt die Stimulationssignale in Abhängigkeit von den Messsignalen. Dieser Betriebsmodus der Vorrichtung 100 wird als "closed loop"-Modus bezeichnet.

Beispielsweise kann, wie in Fig. 1 gezeigt ist, in jedem der Zielgebiete 21 bis 24 eine der Messeinheiten 31 bis 34 platziert sein, welche die neuronale Aktivität des jeweiligen Neuronenverbands 21 bis 24, d.h. das Feuern der beteiligten Neuronen, misst und diese Information an die Generatoreinheit 10 weiterleitet. Alternativ können die Messeinheiten nur in einem Teil der stimulierten Neuronenverbände 21 bis 24 platziert werden oder einige oder alle Messeinheiten können außerhalb der Zielgebiete 21 bis 24 angeordnet werden. Somit kann mittels der Messeinheiten die physiologische Aktivität in einem oder mehreren der stimulierten Zielgebiete und/oder einem oder mehreren damit verbundenen Gebieten gemessen werden.

Die Messeinheiten 31 bis 34 können beispielsweise als Elektroden, insbesondere zur Messung von neuronaler und/oder vegetativer Aktivität, oder als Beschleunigungsmesser ausgeführt sein. Die Anzahl der Messeinheiten ist nicht begrenzt. Es kann nur eine Messeinheit vorgesehen sein, es können aber auch wie in dem Ausführungsbeispiel gemäß Fig. 1 mehrere Messeinheiten im Gehirn und/oder im Bereich des Rückenmarks implantiert werden.

Hinsichtlich des Zusammenwirkens der Generatoreinheit 10 mit den Messeinheiten 31 bis 34 sind verschiedene Ausgestaltungen denkbar. Beispielsweise kann von der Generatoreinheit 10 eine bedarfsgesteuerte Stimulation durchgeführt werden. Hierzu detektiert die Generatoreinheit 10 anhand der von den Messeinheiten 31 bis 34 aufgenommenen Messsignale das Vorhandensein und/oder die Ausprägung eines oder mehrerer krankhafter Merkmale. Beispielsweise kann die Amplitude oder der Betrag der neuronalen Aktivität gemessen werden und dadurch festgestellt werden, ob der betrachtete Rhythmusgenerator eine normale oder eine krankhafte Funktion aufweist. Sobald eine bestimmte Reduktion der rhythmischen Aktivität des Rhythmusgenerators festgestellt wird, kann mit der Stimulation begonnen werden. Beispielsweise kann dazu die rhythmische Aktivität mit einem oder mehreren Schwellwerten verglichen werden. In einer alternativen Ausgestaltung kann die Phasendifferenz der neuronalen Aktivität für die Bedarfssteuerung herangezogen werden. Hierbei werden die Phasendifferenzen der mittels der Messeinheiten 31 bis 34 aufgenommenen neuronalen Aktivitäten der einzelnen Populationen des Rhythmusgenerators bestimmt und bei einer Abweichung der gemessenen Phasendifferenzen von vorgegebenen Werten wird eine Stimulation aktiviert oder die Stimulationsamplitude an die Abweichung angepasst.

Alternativ zum Steuern der Zeitpunkte der Stimulation anhand der von den Messeinheiten 31 bis 34 aufgenommenen Messsignale oder zusätzlich dazu können von der Generatoreinheit 10 anhand der Ausprägung der krankhaften Merkmale Parameter der Stimulationssignale eingestellt werden. Beispielsweise kann die Generatoreinheit 10 anhand der Messsignale die Amplitude der Stimulationssignale oder die Dauer der Stimulation oder die Dauer von Stimulationspulszügen einstellen. Sofern die anhand der aufgenommenen Messsignale festgestellten krankhaften Merkmale während der Stimulation abnehmen, kann die Amplitude der Stimulationssignale verkleinert werden und schließlich gegen Null gehen.

Des Weiteren kann vorgesehen sein, dass die von den Messeinheiten 31 bis 34 aufgenommenen Messsignale direkt oder gegebenenfalls nach einem oder mehreren Verarbeitungsschritten als Stimulationssignale verwendet werden und von der Generatoreinheit 10 in die Stimulationseinheiten 11 bis 14 eingespeist werden. Beispielsweise können die Messsignale verstärkt und gegebenenfalls nach mathematischer Verrechnung (z.B. nach Mischung der Messsignale) mit mindestens einer Zeitverzögerung und linearen und/oder nichtlinearen Verrechnungsschritten und Kombinationen prozessiert und in die Stimulationseinheiten 11 bis 14 eingespeist werden. Der Verrechnungsmodus kann beispielsweise so gewählt werden, dass der krankhaften Reduktion der rhythmischen Aktivität entgegengewirkt wird und das Stimulationssignal mit abnehmender krankhafter neuronaler Aktivität ebenfalls verschwindet oder zumindest deutlich in seiner Stärke reduziert wird.

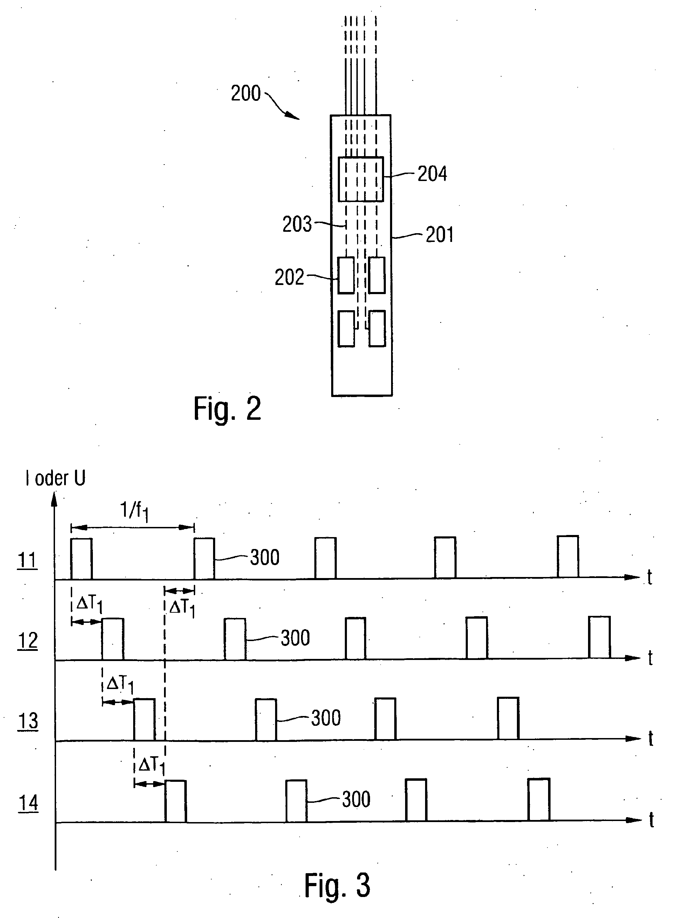

In Fig. 2 ist schematisch eine Elektrode 200 dargestellt, wie sie beispielsweise als Stimulationseinheit 11, 12, 13 oder 14 eingesetzt werden kann. Die Elektrode 200 besteht aus einem isolierten Elektrodenschaft 201 und mindestens einer, beispielsweise zwei oder mehr Stimulationskontaktflächen 202, die in den Elektrodenschaft 201 eingebracht worden sind. Der Elektrodenschaft 201 und die Stimulationskontaktflächen 202 können aus einem biokompatiblen Material hergestellt sein. Ferner sind die Stimulationskontaktflächen 202 elektrisch leitfähig, beispielsweise sind sie aus einem Metall gefertigt, und befinden sich nach der Implantation in direktem elektrischen Kontakt mit dem Nervengewebe. In dem vorliegenden Ausführungsbeispiel kann jede der Stimulationskontaktflächen 202 über eine eigene Zuleitung 203 angesteuert werden bzw. es können über die Zuleitungen 203 die aufgenommenen Messsignale abgeführt werden. Als Alternative können auch zwei oder mehr Stimulationskontaktflächen 202 an dieselbe Zuleitung 203 angeschlossen sein.

Neben den Stimulationskontaktflächen 202 kann die Elektrode 200 eine Referenzelektrode 204 aufweisen, deren Oberfläche größer als die der Stimulationskontaktflächen 202 sein kann. Die Referenzelektrode 204 wird bei der Stimulation des Nervengewebes zur Erzeugung eines Referenzpotentials eingesetzt.

Alternativ kann auch eine der Stimulationskontaktflächen 202 zu diesem Zweck verwendet werden.

Neben ihrer Funktion als eine der Stimulationseinheiten 11 bis 14 kann die Elektrode 200 auch als eine der Messeinheiten 31 bis 34 eingesetzt werden. In diesem Fall werden über mindestens eine der Kontaktflächen 202 Messsignale aufgenommen.

Die Kontaktflächen 202 können mit der Generatoreinheit 10 über Kabel oder über telemetrische Verbindungen verbunden sein.

Ein zur Wiederherstellung der normalen Funktionsweise von Rhythmusgeneratoren geeignetes Stimulationsverfahren, das beispielsweise mittels der Vorrichtung 100 durchgeführt werden kann und bedarfsgesteuert eingesetzt werden kann, ist in Fig. 3 schematisch dargestellt. In Fig. 3 sind untereinander die über die Stimulationseinheiten 11 bis 14 applizierten Stimulationssignale 300 gegen die Zeit t aufgetragen.

Beispielsweise appliziert jede der Stimulationseinheiten 11 bis 14 das Stimulationssignal 300 periodisch an den jeweiligen Neuronenverband 21 bis 24. Die Frequenz f₁, mit welcher die Stimulationssignale 300 pro Stimulationseinheit 11 bis 14 wiederholt werden, kann im Bereich des natürlichen Rhythmus liegen, mit welchem die Neuronen eines einzelnen Neuronenverbandes bei einem gesunden Rhythmusgenerator synchron feuern. Beispielsweise liegt die Frequenz f₁ im Bereich von 0,05 bis 20 Hz, insbesondere im Bereich von 0,05 bis 10 Hz. Unterschiedliche Bewegungsarten können durch unterschiedliche Frequenzen und insbesondere unterschiedliche Phasendifferenzen der einzelnen Neuronenpopulationen des Rhythmusgenerators gekennzeichnet sein. Die Vorrichtung berücksichtigt diese Unterschiede, indem unterschiedliche Programme für die entsprechenden Bewegungsmuster appliziert werden.

Die Verabreichung der Stimulationssignale 300 über die einzelnen Stimulationseinheiten 11 bis 14 erfolgt mit einer zeitlichen Verzögerung zwischen den einzelnen Stimulationseinheiten 11 bis 14. Beispielsweise kann der Beginn zeitlich aufeinander folgender und von unterschiedlichen Stimulationseinheiten applizierten Stimulationssignalen um eine Zeit ΔT₁ verschoben sein.

Im Fall von N Stimulationseinheiten kann die zeitliche Verzögerung ΔT₁ zwischen jeweils zwei aufeinander folgenden Stimulationssignalen 300 beispielsweise im Bereich eines N-tels der mittleren Periode des natürlichen Rhythmus eines einzelnen Neuronenverbands liegen. Da die mittlere Frequenz der rhythmischen Aktivität bei gesunden Rhythmusgeneratoren etwa 0,05 bis 20 Hz beträgt, liegt die zeitliche Verzögerung ΔT₁ beispielsweise im Bereich von 0,05 Sekunde/N bis 20 Sekunde/N. Im günstigsten Fall kann hierdurch eine sofortige Kontrolle der abnormen neuronalen Entladungsmuster in der Zielregion erreicht werden. Des Weiteren kann durch die Stimulation auch ein lang anhaltender synaptischer Umbau in den betroffenen Nervenzellverbänden erzielt werden, sodass die Zielgebiete durch plastische Vorgänge die Fähigkeit zur Ausbildung von Rhythmusgenerator-Aktivität wieder erlernen.

Die zeitliche Verzögerung zwischen zwei aufeinander folgenden Stimulationssignalen 300 braucht nicht notwendigerweise stets gleich groß zu sein. Es kann durchaus vorgesehen sein, dass die Abstände zwischen den einzelnen Stimulationseinheiten 11 bis 14 unterschiedlich gewählt werden. Ferner können die Verzögerungszeiten auch während der Behandlung eines Patienten variiert werden. Auch können die Verzögerungszeiten hinsichtlich der physiologischen Signallaufzeiten adjustiert werden, um dadurch die physiologischen Eigenheiten des stimulierten Rhythmusgenerators zu berücksichtigen.

Durch die Stimulation mittels der Stimulationseinheiten 11 bi.s 14 kann den stimulierten Neuronenverbänden 21 bis 24 der Rhythmus aufgeprägt werden, der durch die Stimulation vorgegeben wird und dem natürlichen Rhythmus entspricht. Nach einer erfolgreichen Stimulation feuern dementsprechend die Neuronen innerhalb jedes der Neuronenverbände 21 bis 24 synchron mit einer im Wesentlichen der Frequenz f₁ entsprechenden Frequenz bzw. einer davon um bis zu ±10% abweichenden Frequenz. Ferner entspricht der Zeitversatz der Aktivität der einzelnen Neuronenverbände 21 bis 24 dem Zeitversatz ΔT₁, mit dem zuvor die Stimulationssignale 300 appliziert worden sind. Folglich entspricht das Muster der von den Neuronenverbänden 21 bis 24 generierten Signale nach einer erfolgreichen Stimulation dem Stimulationsmuster aus Fig. 3.

Zu beachten ist, dass bei den hier beschriebenen Erkrankungen, wie z.B. Schlaganfall, Gait-Ignition-Disorder oder anderen motorischen Störungen, typischerweise keine krankhafte vollständige Synchronisation der Neuronen mehrerer Neuronenverbände vorliegt, d.h. die Neuronen der Neuronenverbände 21 bis 24 feuern typischerweise nicht völlig synchron zu ein und demselben Zeitpunkt. Stattdessen besteht vor der Stimulation beispielsweise nur eine geringe Korrelation zwischen den einzelnen Neuronenverbänden 21 bis 24, es kann sogar vorkommen, dass vor der Stimulation die Neuronen der Neuronenverbände 21 bis 24 in einer unkorrelierten Weise feuern.

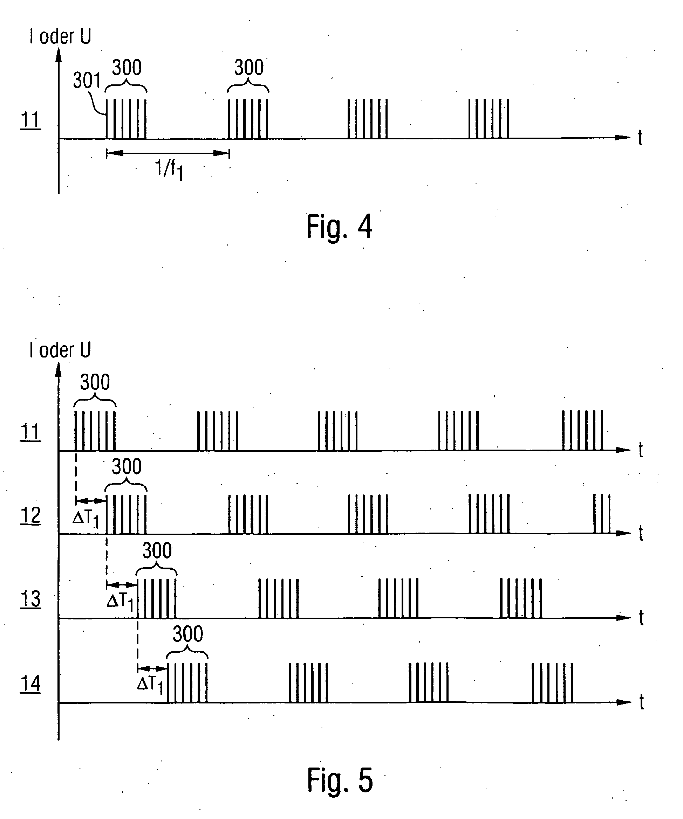

Als Stimulationssignale 300 können beispielsweise strom- oder spannungskontrollierte Pulse verwendet werden. Ferner kann. ein Stimulationssignal 300 ein aus mehreren Einzelpulsen 301 bestehender Pulszug sein, wie er in Fig. 4 anhand der Beispiels der Stimulationseinheit 11 dargestellt ist. Die Pulszüge 300 können jeweils aus 1 bis 100, insbesondere 2 bis 10, elektrischen ladunsgbalancierten Einzelpulsen 301 bestehen. Die Pulszüge 300 werden z.B. als Sequenz mit bis zu 20 oder auch mehr Pulszügen 300 appliziert. Innerhalb einer Sequenz werden die Pulszüge 300 mit der Frequenz f₁ im Bereich von 0,05 bis 20 Hz wiederholt.

Beispielsweise kann die Amplitude der Einzelpulse 301 anhand der mittels der Messeinheiten 31 bis 34 aufgenommenen Messsignale eingestellt werden. Sofern das rhythmische Feuern der untersuchten Neuronenverbände nur schwach oder gar nicht vorhanden ist, wird eine größere Amplitude für die Einzelpulse 301 gewählt. Sobald sich das Verhalten der Neuronenverbände stärker dem normalen, gesunden Verhalten eines Rhythmusgenerators annähert, kann die Amplitude der Einzelpulse 301 reduziert werden.

In Fig. 5 ist das bereits in Fig. 3 gezeigte Stimulationsverfahren mit den Pulszügen 300 als Stimulationssignalen noch einmal dargestellt.

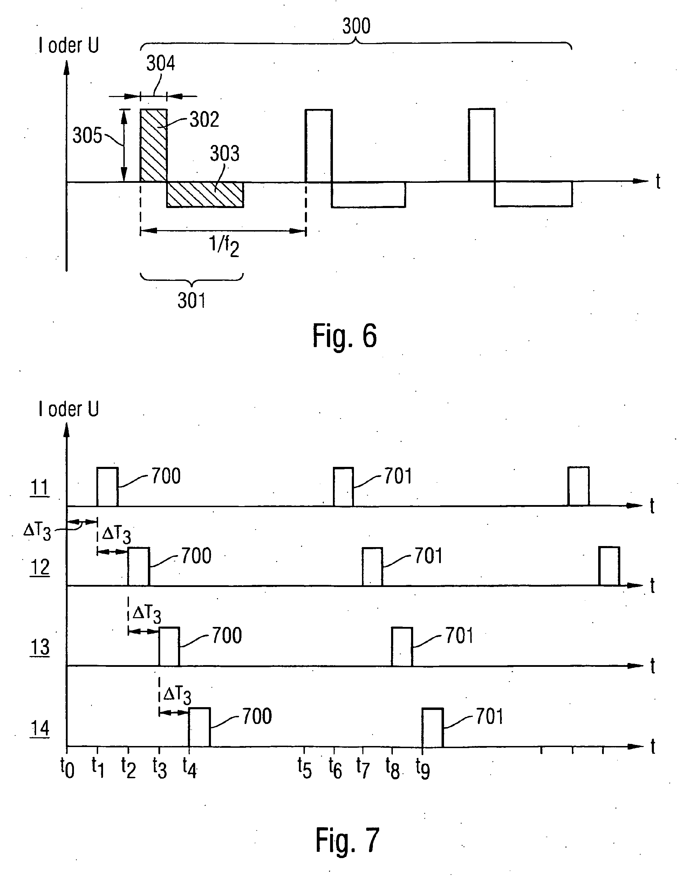

Beispielhaft ist ein Pulszug 300, der aus drei Einzelpulsen 301 besteht, in Fig. 6 gezeigt. Die Einzelpulse 301 werden mit einer Frequenz f₂ zwischen 50 bis 250 Hz, insbesondere oberhalb von 100 Hz, wiederholt. Die Einzelpulse 301 können strom- oder spannungskontrollierte Pulse sein, die sich aus einem anfänglichen Pulsanteil 302 und einem sich daran anschließenden, in entgegengesetzter Richtung fließenden Pulsanteil 303 zusammensetzen, wobei die Polarität der beiden Pulsanteile 302 und 303 gegenüber der in Fig. 6 gezeigten Polarität auch vertauscht werden kann. Die Dauer 304 des Pulsanteils 302 liegt im Bereich zwischen 1 µs und 450 µs. Die Amplitude 305 des Pulsanteils 302 liegt im Falle von stromkontrollierten Pulsen im Bereich zwischen 0 mA und 25 mA und im Fall von spannungskontrollierten Pulsen im Bereich von 0 bis 16 V. Die Amplitude des Pulsanteils 303 ist geringer als die Amplitude 305 des Pulsanteils 302. Dafür ist die Dauer des Pulsanteils 303 länger als die des Pulsanteils 302. Die Pulsanteile 302 und 303 sind idealerweise so dimensioniert, dass die Ladung, welche durch sie übertragen wird, bei beiden Pulsanteilen 302 und 303 gleich groß ist, d.h. die in Fig. 6 schraffiert eingezeichneten Flächen sind gleich groß. Im Ergebnis wird dadurch durch einen Einzelpuls 301 genauso viel Ladung in das Gewebe eingebracht, wie aus dem Gewebe entnommen wird.

Die in Fig. 6 dargestellte Rechteckform der Einzelpulse 301 stellt eine ideale Form dar. Je nach der Güte der die Einzelpulse 301 erzeugenden Elektronik wird von der idealen Rechteckform abgewichen.

Anstelle von pulsförmigen Stimulationssignalen kann die Generatoreinheit 10 beispielsweise auch anders ausgestaltete Stimulationssignale erzeugen, z.B. zeitlich kontinuierliche Reizmuster. Die oben beschriebenen Signalformen und deren Parameter sind nur beispielhaft zu verstehen. Es kann durchaus vorgesehen sein, dass von den oben angegebenen Signalformen und deren Parametern abgewichen wird. Ferner ist es denkbar, dass die Stimulation durch den Patienten erfolgt, beispielsweise durch eine telemetrische Aktivierung. In diesem Fall kann der Patient beispielsweise mittels eines externen Senders die Stimulation für einen vorgegebenen Zeitraum von z.B. 5 Minuten aktivieren oder der Patient kann die Stimulation selbsttätig starten und beenden.

In Fig. 7 ist beispielhaft ein zur Wiederherstellung der normalen Funktionsweise von Rhythmusgeneratoren geeignetes Stimulationsverfahren schematisch dargestellt, das darauf basiert, dass mittels einer oder mehrerer Messeinheiten 31 bis 34 ein Messsignal aufgenommen wird und dieses Messsignal entweder direkt, d.h. ohne weitere Verarbeitungsschritte, oder nach einem oder mehreren Verarbeitungsschritten als Stimulationssignal zur Stimulation der Neuronenverbände verwendet wird. In Fig. 7 sind untereinander die über die Stimulationseinheiten 11 bis 14 applizierten Stimulationssignale gegen die Zeit t aufgetragen.

Bei der vorliegenden Stimulation werden zu einem Zeitpunkt t₀ mittels mindestens einer der Messeinheiten 31 bis 34 ein oder mehrere Messsignale aufgenommen. Die Messsignale können anschließend direkt als Stimulationssignal 700 verwendet werden oder aber zunächst einer Verarbeitung, beispielsweise einer linearen Verarbeitung wie z.B. einer Verstärkung, unterzogen werden und anschließend als Stimulationssignale 700 in die Stimulationseinheiten 11 bis 14 eingespeist werden. Sofern mehr als ein Messsignal aufgenommen werden, können die Messsignale auch miteinander verknüpft werden.

Gemäß einer Ausgestaltung basiert das Stimulationssignal 700 auf einem Pulszug, wie beispielsweise dem in Fig. 4 gezeigten Pulszug 300, dessen Amplitude mit dem Messsignal moduliert wird, und danach in die Stimulationseinheiten 11 bis 14 eingespeist wird.

Das Stimulationssignal 700 wird in die unterschiedlichen Stimulationseinheiten 11 bis 14 zeitverzögert eingespeist. In Fig. 7 sind die Startzeitpunkte der jeweiligen Stimulationen mit t₁ bis t₄ bezeichnet. Die Zeitpunkte t₁ bis t₄ sind von dem jeweils vorangehenden Zeitpunkt t₀ bis t₃ um ein Zeitintervalle ΔT₃ verzögert. Das Zeitintervall ΔT₃ kann analog zu dem im Zusammenhang mit Fig. 3 beschriebenen Zeitintervall ΔT₁ gewählt werden, d.h. es können Verzögerungszeiten im Bereich von 0,05 Sekunde/N bis 20 Sekunde/N gewählt werden, wobei N die Anzahl der Stimulationseinheiten angibt.

Nach der Stimulation mittels des Stimulationssignals 300 kann eine erneute Stimulation erfolgen. Dazu kann das nächste Messsignal beispielsweise bereits zum Zeitpunkt t₄ aufgenommen werden, es kann aber auch, wie in Fig. 7 dargestellt ist, erst zu einem späteren Zeitpunkt t₅ aufgenommen werden. Aus dem zu dem Zeitpunkt t₅ ermittelten Messdaten wird ein neues Stimulationssignal 701 generiert und zu Zeitpunkten t₆ bis t₉ in die Stimulationseinheiten 11 bis 14 eingespeist. Der Abstand zwischen den Zeitpunkten t₅ bis t₉ beträgt wieder jeweils ΔT₃.

Entsprechend kann mit der Stimulation fortgefahren werden. Die Stimulation kann entweder nach einer bestimmten Anzahl von Zyklen beendet bzw. unterbrochen werden oder es kann anhand der Messsignale geprüft werden; ob eine ausreichende rhythmische Aktivität der Neuronenverbände mittels der Stimulation erzielt wurde und dementsprechend die Stimulation fortgeführt oder beendet werden. Wie bei der in den Fig. 3 und 5 gezeigten Stimulation soll auch durch die Stimulation gemäß Fig. 7 den stimulierten Neuronenverbänden 21 bis 24 der Rhythmus aufgeprägt werden, der durch die Stimulation vorgegeben wird und dem natürlichen Rhythmus entspricht.

Gemäß einer Ausgestaltung beträgt die Zeitspanne zwischen zwei aufeinander folgenden Stimulationssignalen nicht stets ΔT₃, sondern die Zeitabstände zwischen den einzelnen Stimulationseinheiten 11 bis 14 werden unterschiedlich gewählt und/oder werden während der Behandlung des Patienten justiert. Dadurch können z.B. physiologische Signallaufzeiten des stimulierten Rhythmusgenerators berücksichtigt werden.

Eine weitere Alternative zu dem oben beschriebenen Stimulationsverfahren kann darin bestehen, dass die Messsignale nicht zu einem einzigen Zeitpunkt aufgenommen werden, sondern während eines Zeitraums. Dieser Zeitraum kann z.B. der Periodendauer der normalen rhythmischen Aktivität entsprechen. Beispielsweise kann aus den während dieses Zeitraums aufgenommenen Signalen die Varianz gebildet werden und entweder direkt oder nach einer weiteren Verarbeitung als Stimulationssignal verwendet werden.

Wie weiter oben bereits beschrieben wurde, kann der Abstand zwischen zwei aufeinander folgenden Stimulationen mittels derselben Stimulationseinheit grundsätzlich frei gewählt werden. Für den Abstand kann beispielsweise die Zeitspanne N x ΔT₃ oder ein ganzzahliges Vielfaches davon gewählt werden, damit die Stimulationssignale den stimulierten Neuronenverband stets mit der gleichen Phase stimulieren.

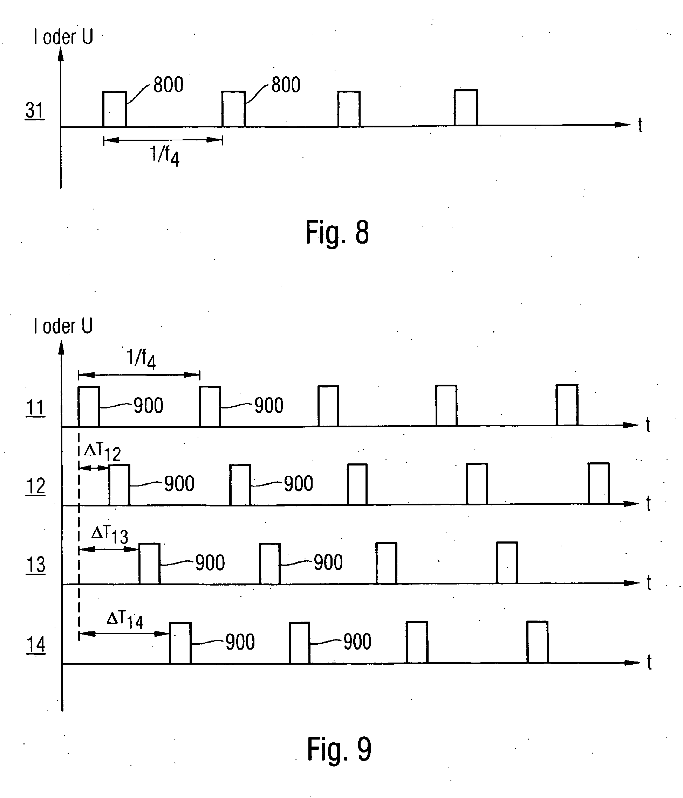

Weitere Stimulationsformen werden nachfolgend anhand der Fig. 8 und 9 erläutert. In Fig. 8 sind Messsignale 800 gegen die Zeit t aufgetragen. Die Messsignale 800 sind beispielsweise mittels der Messeinheit 31, die in den Neuronenverband 21 implantiert worden ist, aufgenommen worden. Fig. 8 zeigt, dass die Neuronen des Neuronenverbands 21 beispielsweise mit einer gewissen Periodizität elektrische Signale generieren. Die Messsignale 800 können die Form von Bursts haben, innerhalb derer die von den beteiligten Neuronen erzeugten Signale angeordnet sind. Die Bursts 800 werden mit einer Frequenz f₄ im Bereich von 0,05 bis 20 Hz wiederholt.

Die Messsignale 800 können entweder in unveränderter Form in die Stimulationseinheiten 11 bis 14 eingespeist werden oder zunächst Verarbeitungsschritten unterzogen werden und danach als Stimulationssignale verwendet werden. Beispielsweise können die Messsignale 800 mittels eines Bandpass- oder Tiefpassfilters gefiltert werden und eventuell verstärkt werden, bevor sie als Stimulationssignale genutzt werden. Ferner können die Messsignale 800 verzögert in die einzelnen Stimulationseinheiten 11 bis 14 eingespeist werden. Eine solche Stimulationsform ist schematisch in Fig. 9 dargestellt. Dort ist gezeigt, dass Stimulationssignale 900, die aus den.Messsignalen 800 gewonnen wurden, zeitversetzt in die Stimulationseinheiten 11 bis 14 eingespeist werden.

Die Stimulationssignale 900 können z.B. generiert werden, indem die Messsignale 800 mittels eines Band- oder Tiefpassfilters gefiltert werden und mit den daraus erhaltenen Signalen kurze Pulszüge, wie z.B. den in Fig. 4 dargestellten Pulszügen 300, amplitudenmoduliert werden. Dies hat zur Folge, dass die Stimulationssignale 900 die gleiche Periodenlänge 1/f₄ wie die Messsignale 800 aufweisen. In die Stimulationseinheit 11, die genauso wie die Messeinheit 31 in den Neuronenverband 21 implantiert ist, können die Stimulationssignale 800 phasengleich mit den Messsignalen.800 eingespeist werden. Dies kann zur Folge haben, dass die zeitgleich mit den von den Neuronen generierten Bursts 800 applizierten Stimulationssignale 900 den natürlichen Rhythmus des Neuronenverbands 21 stabilisieren.

Die Stimulationssignale 900 können des Weiteren mit den in Fig. 9 gezeigten Verzögerungen ΔT₁₂, ΔT₁₃ bzw. ΔT₁₄ über die Stimulationseinheiten 12 bis 14 an die Neuronenverbände 22 bis 24 appliziert werden. Dadurch kann der natürliche Rhythmus der Neuronenverbände 22 bis 24 stabilisiert werden oder dieser kann, sofern die betroffenen Neuronenverbände vor der Stimulation keinen oder einen nicht normalen Rhythmus aufwiesen, in diese Neuronenverbände induziert werden.

Die Verzögerungen ΔT₁₂, ΔT₁₃ und ΔT₁₄ können entweder wie oben beschrieben frei gewählt werden oder sie können durch Messungen ermittelt werden. Beispielsweise kann ein Anregungssignal, z.B. ein pulsförmiges Signal, von der Stimulationseinheit 11 appliziert werden. Danach werden mittels der Messeinheiten 32 bis 34 Antwortsignale aufgenommen und die Zeiten, die zwischen dem Anregungssignal und den Antwortsignalen jeweils vergehen, werden als Verzögerungszeiten ΔT₁₂, ΔT₁₃ und ΔT₁₄ für die jeweilige Stimulationseinheit 12 bis 14 herangezogen. Im Ergebnis kann durch diese Art der Stimulation in die Neuronenverbände 21 bis 24 eine rhythmische Aktivität induziert werden, welche dem Stimulationsmuster, wie es in Fig. 9 gezeigt ist, gleicht oder zumindest ähnelt und welche der normalen, gesunden rhythmischen Aktivität des stimulierten Rhythmusgenerators entspricht.

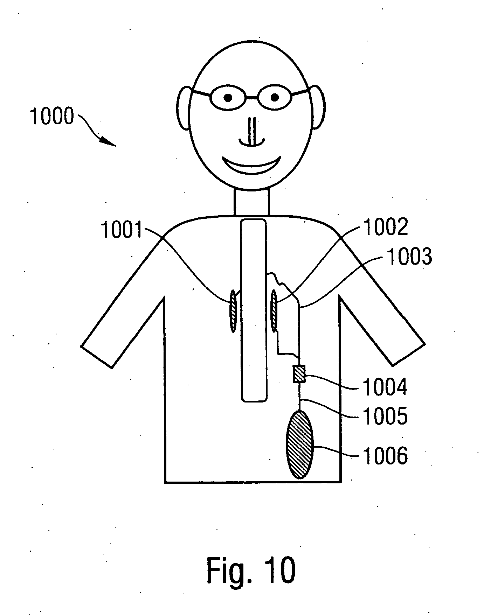

In Fig. 10 ist eine Vorrichtung 1000 zur Wiederherstellung der normalen Funktionsweise von Rhythmusgeneratoren mittels Stimulation von Neuronen während ihres bestimmungsgemäßen Betriebs dargestellt. Dazu sind Stimulationselektroden 1001 und 1002 im Bereich des Rückenmarks eines Patienten implantiert worden. Jede der Stimulationselektroden 1001 und 1002 steht mit einem unterschiedlichen Neuronenverband in Verbindung, die zu demselben Rhythmusgenerator gehören. Ferner verfügt die Vorrichtung 1000 über mindestens einen Sensor, der beispielsweise in die Stimulationselektroden 1001 und/oder 1002 integriert ist. Die Stimulationselektroden 1001 und 1002 sind jeweils mit einem Elektrodenkabel 1003 über einen Konnektor 1004 und ein Verbindungskabel 1005 mit einer Generatoreinheit 1006 verbunden. Alle Teile der Vorrichtung 1000 sind im Körper des Patienten implantiert. Die Generatoreinheit 1006 kann eine Steuerelektronik enthalten, welche die Stimulationsverfahren realisiert. Als Energiequelle kann die Generatoreinheit 1006 über eine langlebige Batterie oder einen aufladbaren Akkumulator verfügen. Die Generatoreinheit 1006 kann beispielsweise subkutan im Bereich des Unterbauchs des Patienten platziert sein. In einer alternativen Ausgestaltung kann die Generatoreinheit 1006 ein Halbimplantat mit einer außerhalb des Körpers befindlichen Energiequelle sein. Die Generatoreinheit 1006 kann über eine Sicherheitsschaltung verfügen, die bewirkt, dass dem Fachmann bekannte Sicherheitsgrenzen, wie z.B. ein maximal verträglicher Ladungseintrag, eingehalten werden.

## Patentansprüche

1. Vorrichtung (100; 1000) umfassend:
- mindestens eine Messeinheit (31-34) zum Aufnehmen von Messsignalen von Neuronen,
- eine Generatoreinheit (10) zum Erzeugen von elektrischen Stimulationssignalen in Abhängigkeit von den Messsignalen, wobei die Stimulationssignale Sequenzen von Pulszügen sind und die Pulszüge mit einem aus den Messsignalen gewonnenem Modulationssignal moduliert sind, und
- eine Mehrzahl von mit der Generatoreinheit (10) verbundenen Stimulationseinheiten (11-14), wobei die Generatoreinheit derart konfiguriert ist, daß Stimulationseinheiten (11-14) mit den Stimulationssignalen eine Mehrzahl von Neuronenverbänden jeweils zeitversetzt stimulieren und dadurch den stimulierten Neuronenverbänden eine zeitlich verschobene Aktivität induzieren, wobei die Stimulationssignale von jeder der Stimulationseinheiten (11-14) mit einer Frequenz f₁ appliziert werden,
**dadurch gekennzeichnet,**
- **dass** die Anzahl der Stimulationseinheiten (11-14) N beträgt und die Verzögerung zwischen den von den Stimulationseinheiten (11-14) applizierten Stimulationssignalen im Mittel im Wesentlichen einem Term 1/(f₁ x N) entspricht.

2. Vorrichtung (100; 1000) nach Anspruch 1, wobei die Generatoreinheit (10) in Abhängigkeit von den Messsignalen entscheidet, ob eine Stimulation mittels der Stimulationseinheiten (11-14) durchgeführt wird.

3. Vorrichtung (100; 1000) nach Anspruch 1 oder 2, wobei die Generatoreinheit (10) in Abhängigkeit von den Messsignalen einen Parameter der Stimulationssignale bestimmt.

4. Vorrichtung (100; 1000) nach einem der vorhergehenden Ansprüche, wobei die Generatoreinheit (10) die Stimulationssignale in Abhängigkeit eines Vergleichs der Messsignale mit einem oder mehreren vorgegebenen Schwellwerten erzeugt.

5. Vorrichtung (100; 1000) nach einem der vorhergehenden Ansprüche, wobei die Frequenz f₁ einer Frequenz entspricht, mit der ein Neuronenverband eines gesunden Rhythmusgenerators elektrische Signale erzeugt.

6. Vorrichtung (100; 1000) nach einem der vorhergehenden Ansprüche, wobei die stimulierten Neuronenverbände Teil eines Rhythmusgenerators sind.

## Claims

1. An apparatus (100; 1000) comprising:
- at least one measuring unit (31-34) for recording measured signals of neurons;
- a generator unit (10) for producing electrical stimulation signals in dependence on the measured signals, wherein the stimulation signals are sequences of pulse trains and the pulse trains are modulated using a modulation signal obtained from the measured signals; and
- a plurality of stimulation units (11-14) connected to the generator unit (10), wherein the generator unit is configured such that stimulation units (11-14) stimulate a plurality of neural assembles with a respective time offset using the stimulation signals and thereby induce a time-displaced activity in the stimulated neural assemblies, wherein the stimulation signals have a frequency f₁ applied by each of the stimulation units (11-14),
**characterised in that**
- the number of stimulation units (11-14) amounts to N and the delay between the stimulation signals applied by the stimulation units (11-14) substantially corresponds on average to a term 1/(f₁ x N).

2. An apparatus (100; 1000) in accordance with claim 1, wherein the generator unit (10) decides in dependence on the measured signals whether a stimulation is carried out by means of the stimulation units (11-14).

3. An apparatus (100; 1000) in accordance with claim 1 or claim 2, wherein the generator unit (10) determines a parameter of the stimulation signals in dependence on the measured signals.

4. An apparatus (100; 1000) in accordance with any one of the preceding claims, wherein the generator unit (10) produces the stimulation signals in dependence on a comparison of the measured signals with one or more predefined threshold values.

5. An apparatus (100; 1000) in accordance with any one of the preceding claims, wherein the frequency f₁ corresponds to a frequency with which a neural assembly of a healthy rhythm generator produces electrical signals.

6. An apparatus (100; 1000) in accordance with any one of the preceding claims, wherein the stimulated neural assemblies are part of a rhythm generator.

## Revendications

1. Appareil (100 ; 1000) comprenant :
- au moins une unité de mesure (31-34) pour enregistrer des signaux de mesure provenant de neurones,
- une unité à générateur (10) pour engendrer des signaux de stimulation électrique en fonction des signaux de mesure, lesdits signaux de stimulation étant des séquences de trains d'impulsions et les trains d'impulsions sont modulés avec un signal de modulation récupéré à partir des signaux de mesures, et
- une pluralité d'unités de stimulation (11-14) connectées à l'unité à générateur (10), dans lequel l'unité à générateur est configurée de telle façon que les unités de stimulation (11-14) stimulent avec les signaux de stimulation une pluralité de groupes de neurones respectivement de manière décalée dans le temps et induisent ainsi dans les groupes de neurones stimulés une activité temporellement déplacée, et les signaux de stimulation sont appliqués par chacune des unités de stimulation (11-14) avec une fréquence f₁,
**caractérisé en ce que**
- le nombre des unités de stimulation (11-14) est égal à N et le retard entre les signaux de stimulation appliquée par les unités de stimulation (11-14) correspond en moyenne sensiblement à un terme 1/(f₁ x N).

2. Appareil (100 ; 1000) selon la revendication 1, dans lequel l'unité à générateur (10) décide en fonction des signaux de mesure si une stimulation est exécutée au moyen des unités de stimulation (11-14).

3. Appareil (100 ; 1000) selon la revendication 1 ou 2, dans lequel l'unité à générateur (10) détermine un paramètre des signaux de stimulation en fonction des signaux de mesure.

4. Appareil (100 ; 1000) selon l'une des revendications précédentes, dans lequel l'unité à générateur (10) engendre les signaux de stimulation en fonction d'une comparaison des signaux de mesure avec une ou plusieurs valeurs seuil prédéterminées.

5. Appareil (100 ; 1000) selon l'une des revendications précédentes, dans lequel la fréquence f₁ correspond à une fréquence avec laquelle un groupe de neurones engendre des signaux électriques d'un générateur de rythme sain.

6. Appareil (100 ; 1000) selon l'une des revendications précédentes, dans lequel les groupes de neurones stimulés font partie d'un générateur de rythme.
